Europäisches Patentamt

European Patent Office

Office européen des brevets

(19)

(11) **EP 0 745 612 B1**

(12) **EUROPEAN PATENT SPECIFICATION**

(45) Date of publication and mention
of the grant of the patent:
**07.11.2001 Bulletin 2001/45**

(51) Int Cl.⁷: **C07K 16/42, A61K 39/395**

(21) Application number: **96107651.0**

(22) Date of filing: **14.05.1996**

(54) **Anti-idiotypic antibodies which induce an immune response against epidermal growth factor receptor**

Antiidiotypische Antikörper die eine Immunantwort gegen den Rezeptor für epidermalen Wachstumsfaktor induzieren

Anticorps anti-idiotypiques qui induisent une réponse immunitaire contre le récepteur de croissance épidermique

(84) Designated Contracting States:
**AT BE CH DE DK ES FR GB GR IE IT LI LU NL PT SE**
Designated Extension States:
**LT LV SI**

(30) Priority: **26.05.1995 EP 95107967**

(43) Date of publication of application:
**04.12.1996 Bulletin 1996/49**

(73) Proprietor: **MERCK PATENT GmbH
64293 Darmstadt (DE)**

(72) Inventors:
• **Carceller, Ana
08028 Barcelona (ES)**
• **Rosell, Elisabet
08008 Barcelona (ES)**
• **Gomez, Alicia
08029 Barcelona (ES)**
• **Adan, Jaume
28 Mataro (ES)**
• **Piulats, Jaume
08030 Barcelona (ES)**

(56) References cited:
**EP-A- 0 586 002        WO-A-92/15683**

• **TUMOUR BIOLOGY, vol. 15, no. 4, 1994, BASEL, SWITZERLAND, pages 188-202, XP000579858 O. MERIMSKY ET AL.: "Antigens and antibodies in malignant melanoma."**
• **PROTEIN ENGINEERING, vol. 4, no. 7, October 1991, OXFORD, GB, pages 773-783, XP002012218 C. KETTLEBOROUGH ET AL.: "Humanization of a mouse monoclonal antibody by CDR-grafting: the importance of framework residues on loop conformation."**
• **CANCER RESEARCH, vol. 47, no. 14, 15 July 1987, BALTIMORE, MD, USA, pages 3692-3696, XP002012219 U. RODECK ET AL.: "Tumor growth modulation by a monoclonal antibody to the epidermal growth factor receptor: immunologically mediated and effector cell-independent effects."**
• **IMMUNOLOGIA, vol. 12, 1993, MILANO, ITALY, page 122 XP002012220 E. SUAREZ ET AL.:**
• **THE JOURNAL OF IMMUNOLOGY, vol. 150, no. 2, 15 January 1993, BALTIMORE, MD, USA, pages 508-516, XP002012221 M. TSUJISAKI ET AL.: "The analysis of internal image-bearing anti-idiotypic monoclonal antibody in relation to carcinoembryonic antigen."**
• **CELLULAR AND MOLECULAR IMMUNOLOGY, vol. 41, no. 1, February 1995, NEW YORK, NY, USA, pages 179-184, XP002012222 K. STEINER ET AL.: "Distribution of humanized mab 425 (EMD 62 000) in rats and specific localization in tumour-bearing nude mice."**

**Description**

**1. Field of the Invention**

The present invention is directed to anti-idiotypic antibodies which induce an immune response against tumors which bear as antigen the epidermal growth factor receptor (EGFR). This invention relates, above all, to anti-idiotypic antibodies which have the internal image of the antigen and are capable to mimick the external domain of human EGFR. As preferred embodiments of the invention, said antibodies derive from the murine mAb 425 or from its humanized and chimeric versions. The antibodies of the invention can be used for tumor immunotherapy and immunoprophylaxis.

**2. Backround of the Invention**

The specification relates to several abbreviations and technical terms which are defined as follows:

"FRs" (framework regions) mean the four subregions of the light or heavy chain variable regions that support the three CDRs.
"CDRs" (complementarity determining regions) mean the three subregions of the light or heavy chain variable regions which have hypervariable sequences and form loop structures that are primarily responsible for making direct contact with antigen.
"EGF" and "EGFR" mean the epidermal growth factor ant its receptor.
"PCR" means the polymerase chain reaction.
"scFv" means single-chain Fv which is an antibody fragment.
"$V_L$" means light chain variable region.
"$V_k$" means kappa light chain variable region.
"$V_H$" means heavy chain variable region.
"Chimeric" or partially humanized antibodies mean antibodies comprising constant regions deriving from human sources and variable regions (CDRs included) deriving from non-human sources, e.g. from the mouse.
"Humanized" or fully humanized antibodies mean antibodies comprising constant regions and FRs deriving from human sources whereas the CDRs derive from non-human sources.
"Ab1" means the first or parent antibody which induces a cascade of succession antibodies by immunisation.
"Ab2" means the typical anti-idiotypic antibody (= "anti-id") which is directed to the idiotypes of the Ab1.
"Ab3" means an anti-idiotypic antibody which is directed against the idiotypes of the Ab2, and thus, is comparable with Ab1.
PBS means phosphate buffered saline
FCS means fetal calf serum
HBSS means Hanks balanced salt solution
FITC means fluoresceineisothiocyanate
MTC means mixed cell culture
KLH means Keyhole Limpet Hemocyanine
CFA means complete Freund's adjuvants
HRPO means human recombinant peroxidase

Anti-idiotypic antibodies (anti-idotypes or "anti-ids") are antibodies directed against epitopes (called idiotypes) located in the antigen binding region or variable region of another antibody molecule. The interactions between idiotypes (Ab1) and anti-idiotypes (Ab2) play an important role in the maintenance of immune homeostasis. A network of idiotypes and anti-idiotypes has beeb invoked to explain immunregulation (Jerne, F.G.,1974, *Ann.Immunol.125C:373)*. The "internal image" anti-idiotypic antibodies mimic the three-dimensional structure of the antigen recognized by the Ab1. The administration of Ab2 can induce the production of Ab3 antibodies against the original antigen. Idiotype vaccines have been applied successfully on several transmissible diseases and in the treatment of cancer ( for example:
Uytdehaag, F.G., and C.M.H. Osterhaus, 1986, *J.Immunol.134:1225;* Kennedy, R.C. et al. 1986, *Science 232: 220;* Hiernaux, J.R, 1988; *56:1407;* Stein, K.E., and T. Soderstrom, 1984, *J.Exp.Med. 160:1001)*. In tumor immunology, the idiotypic vaccination has been used to modulate tumor growth in experimental in vivo and in vitro systems (Smorodinsky, N.I. et al, 1988; *Eur. J.Immunol. 18:1713.* Viale,G., et al., 1987, *J.Immunol. 139:4250)*. Herlyn et al (1987, *Proc. Natl. Acad. Sci. USA. 76:1438)* have reported promising results in a clinical trial on patients with advanced colorectal carcinoma, arrest of metastases and partial clinical remissions were observed in patients treated only with anti-ids. Anti-idiotypic antibodies and its use for tumor therapy are also known from US 4,918,164 and EP 0141 783, for example. EP-A-0 586 002 describes monoclonal antibodies recognizing EGFR and discloses pharmaceutical compositions comprising this antibody or a corresponding monoclonal anti-idiotypic antibody.

Epidermal growth factor (EGF) is a polypeptide hormone which is mitogenic for epidermal and epithelial cells. When EGF interacts with sensitive cells, it binds to membrane receptors (EGFR). The EGFR is a transmembrane glycoprotein of about 170 kD and is a gene product of the c-erb-B proto-oncogene.

MAb 425 is a murine monoclonal antibody raised against the well known human A431 carcinoma cell line (ATCC CRL 1555), binds to a polypeptide epitope of the external domain of the human EGFR, and inhibits the binding of EGF. MAb 425 (ATCC HB 9629) was found to mediate tumor cytotoxicity in vitro and to suppress tumor cell growth of epidermoid and colorectal carcinoma-derived cell lines in vitro (Rodeck et al., *Cancer Res.* 1987. *47:* 3692). Humanized and chimeric versions of MAb 425 have been disclosed in WO 92/15683.

The binding of the EGF to the receptor activates several biochemical processes leading to DNA replication and cell division (Carpenter, G,1987, *Annu.Rev. Biochem. 56:881*). The EGFR system has been recently implicated in oncogenic transformation of cells (DiFiore, P.P. et al., 1987, *Cell. 51:1063*.10). An autocrine stimulated cell proliferation has been proposed for several tumors which express the EGFR and secrete EGF or TGFα (Ennis,B.W. et al., 1989, *Mol. Endo. 3:1830*), higher expression of EGFR in cell surface has been detected in tumors of diverse tissue origin: breast, bladder, carcinomas, melanomas and non-neuronal brain tumors (Neal, D.E. et al., 1985; *Lancet 1: 366;* Libermann,T.A. et al., 1984, *Cancer Res. 44: 753*; Herlyn,M. et al., 1982, *J.Clin.Immunol.2: 135*). EGFR a therapeutical target because it is directly implicated in cellular proliferation of several tumors. Its overexpression has shown to be of bad prognostic value, correlating with an augmented invasive potential (Sainsbury,J.R et al.,1985, *Lancet 1:364*).

The passive or active immunization against EGFR in cancer patients leads to circulating specific antibodies which act as antagonists of EGF and TGFα, blocking their access to the receptor and inhibiting tumor growth. EGFR is a good candidate for an Idiotype vaccine since it can be purified from tumor cells but is not available in amount sufficient for therapeutic purposes.

Anti-idiotypic antibodies, in general, and their structural comparison with their antigens, and internal images, were investigated and discussed by several goups (e.g. Tsjisaki, M. et al., 1993, J Immunol. 150:508; Raychaudhuri, s. at al., 1990, J. Immunol. 145:760; Bruck, C., et al., 1986, Proc.Natl.Acad.Sci. USA 83:6578.

## 3. Summary of the Invention

The present invention provides for the first time new anti-idiotypic antibodies (anti-ids) which induce an immune response against the epidermal growth factor receptor. Especially, the invention provides at least two such anti-ids (designated as 5A6 and 3B6) which mimick an antigen site on the surface of the human epidermal growth factor receptor. Thus, the antibodies according to the invention, especially those that can mimick the EGFR can be used to induce and to increase the immune response against all kinds of human tumors which express the epidermal growth factor receptor on their surface, melanomas, gliomas and carcinomas included. The said antibodies can be used also for diagnostic applications regarding locating and assessing the said tumors *in vitro* or *in vivo*.

Thus, it is an object of the invention to provide a new antibody which is a monoclonal anti-idiotypic (Ab2) antibody inducing an immune response against epidermal growth factor receptor (EGFR).

In detail, the invention relates to a monoclonal anti-idiotypic antibody mimicking the internal image of EGFR antigen recognized by a corresponding murine, humanized or chimeric, monoclonal idiotypic (Ab1) antibody. This Ab1 antibody can be a non-human, especially a murine, a humanized or a chimeric monoclonal antibody as defined above. Since it is imaginable that there are per se several Ab1 antibodies directed against the EGFR epitope the invention relates also to anti-idiotypic antibodies which can recognize this group of Ab1 anti-EGFR antibodies.

The invention relates, furthermore, to monoclonal anti-idiotypic anti-EGFR antibodies which can be obtained from the corresponding Ab1 anti-EGFR antibody, for example, by immunisation.

Therefore, it is an object of the present invention to provide a monoclonal anti-idiotypic antibody which is obtainable by immunisation of an animal with a corresponding murine, humanized or chimeric, idiotypic (Ab1) antibody.

The anti-idiotypic antibodies of the invention are perferably obtained by immunisation with the monoclonal anti-EGFR antibody 425 or humanized and chimeric versions thereof. MAb 425 is produced by the known cell line deposited under ATCC HB 9629.

Thus it is an object of the invention to provide a monoclonal antibody characterized in that the idiotypic (Ab1) antibody is mAb 425 (ATCC HB 9629) or derives from it by humanisation or chimerisation according to known methods, for example, as described in WO 92/15683.

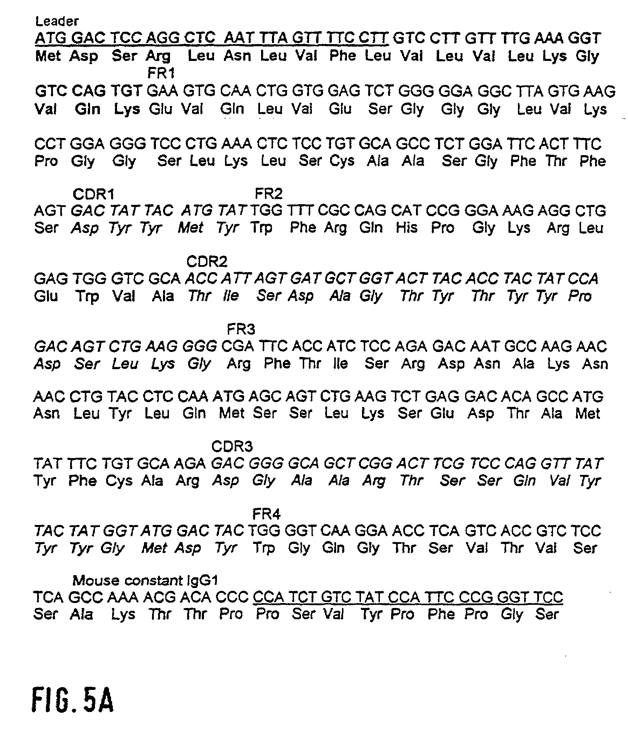

The invention relates also to specific anti-idiotypic antibodies which have well defined amino acid sequences in the hypervariable (CDRs) and variable (FRs) regions of the antibodies and which are indicated in *Fig.5A-F.*

Therefore, it is another object of the invention to provide an anti-idiotypic monoclonal antibody wherein the CDR regions and the FR regions of said antibody comprise the amino acid sequences of *Fig. 5A-F*. Moreover, it is an object of the invention to provide, as preferred embodiment, an anti-idiotypic antibody comprising an amino acid or a nucleotide sequence of Fig. 5A-F.

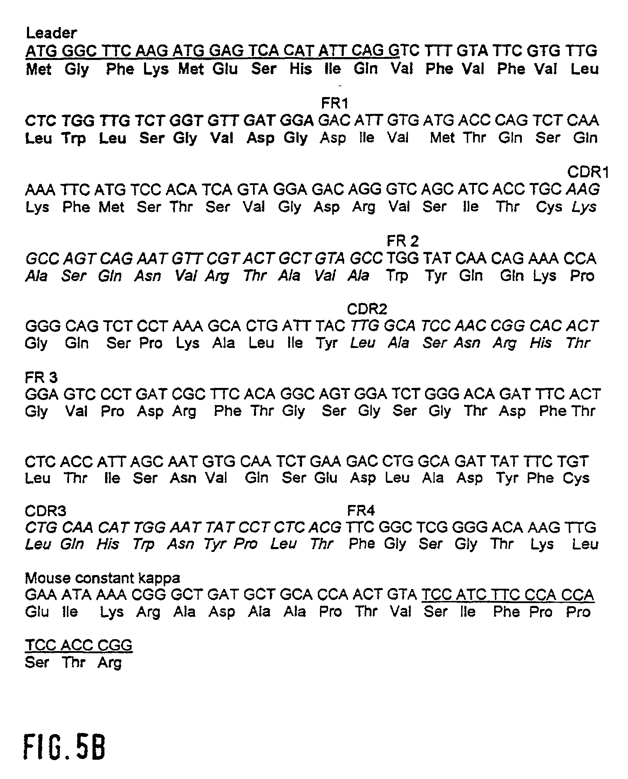

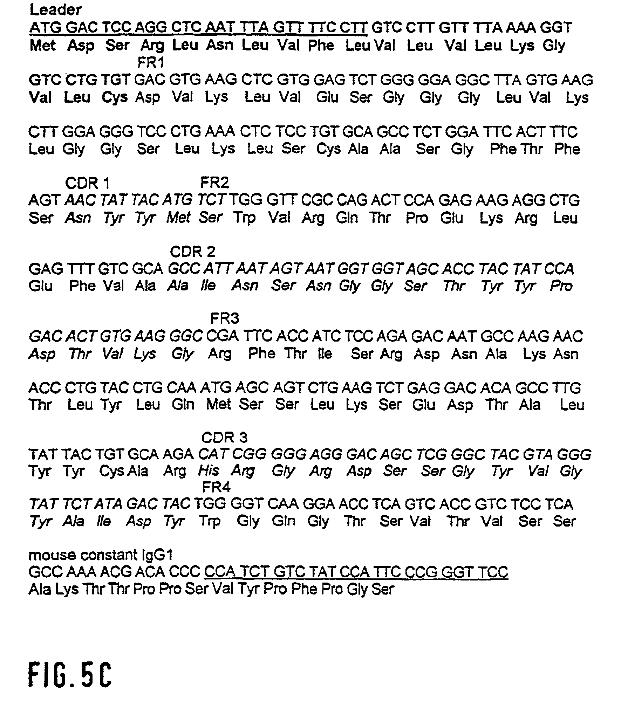

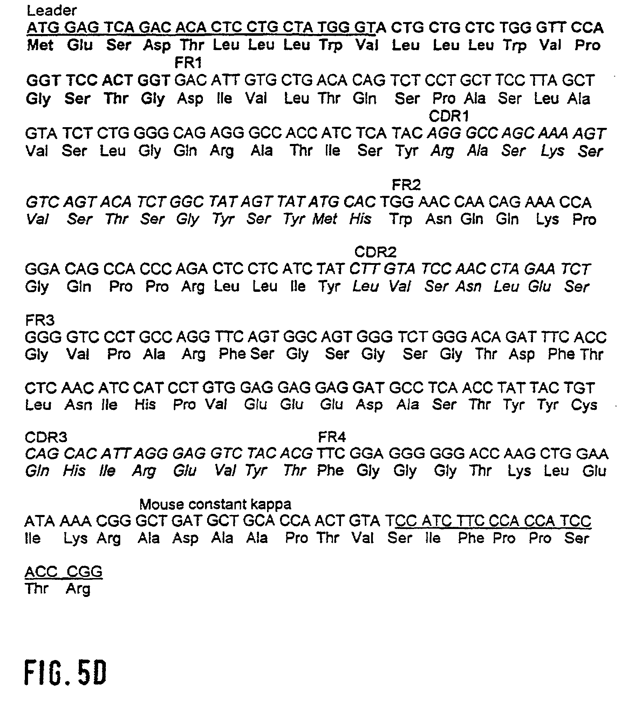

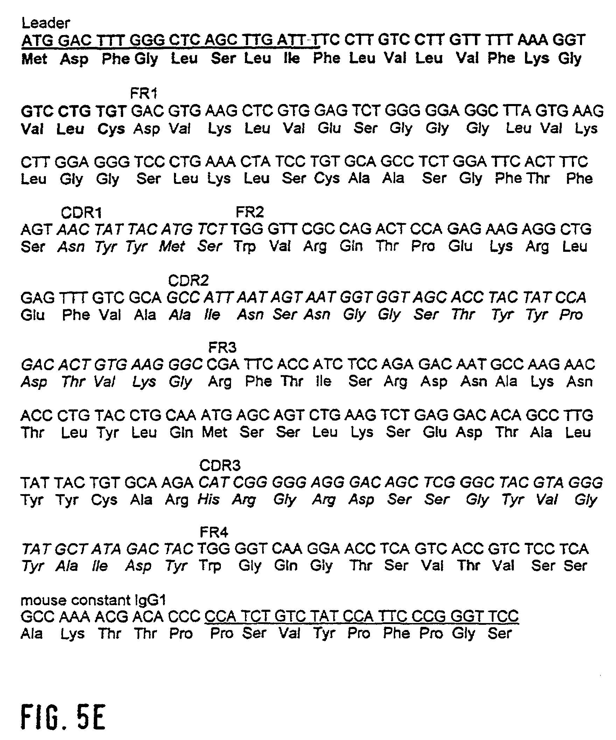

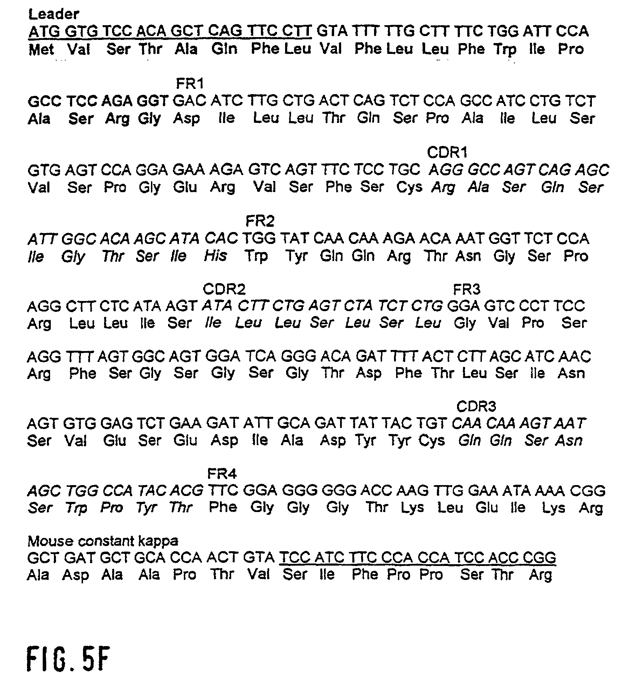

According to the invention, the disclosed sequences comprise also variations and changes which derive from the exactly specified sequences of Figure 5 by spontaneous or induced chemically or physically caused mutations, insertions, deletions and replacements of single amino acid or nucleotide residues, provided that the biological activity and properties of the final antibodies have not been essentially altered.

The term anti-idiotypic antibody also includes parts or fragments of such an antibody, for example, single chain Fv molecules or F(ab)'$_2$ and Fab' molecules which are well known in the art (Skerra and Plückthun, *Science* 1988. *240*: 1038; Better et al., *Science* 1988. *240*: 1041) Single-chain Fvs (wherein the V$_L$ and the V$_H$chain are linked together) have been also described (e.g.: Bird et al., *Science* 1988. *242*: 423; Huston et al., *Proc. Natl. Acad. Sci. USA* 1988. *85*: 5879).

The invention relates, moreover, to a process for the preparation of an anti-idiotypic antibody as defined in the specification and in the claims, characterized in that an animal is immunized with a corresponding idiotypic (Ab1) antibody, which binds to the antigen directly by its CDR regions, the synthesized anti-idiotypic (Ab2) antibody, which binds to the idiotypes of the Ab1 antibody, is separated and purified by convenient methods.

Furthermore, it is an object of the invention to provide a pharmaceutical composition comprising an anti-idiotypic monoclonal antibody as defined in the specification and, optionally, a pharmaceutically acceptable carrier.

Finally, the invention relates to the use of said anti-idiotypic antibody for the manufacture of a drug directed against tumors.

The present invention describes the obtention of three monoclonal anti-idiotypic antibodies (5A6, 3B6 and 15H8) that recognize idiotypes overlapping with the antigen-binding site (paratope) of mAb 425. The serologic and immunologic studies have shown that 5A6 and 3B6 carry the internal image of the epitope recognized by mAb 425 inducing a humoral response in syngeneic (Balb/c mice) and allogeneic (B6D2F1 mice) system. True internal image Anti-Ids would act as antigen surrogates in animals of different species, however these Anti-Ids have been ineffective to generate Ab1-like response in rabbits and rats. The cloning and sequencing of their V regions has shown the existence of amino acid homologies between CDRs of both light and heavy chains and some EGFR residues located in the ligand binding domain, suggesting an antigen mimicry. The study of antigenicity of homologous regions suggest a possible explanation for the lack of biological effect in heterogeneic system and supports a potential effectivity in human therapy.

### 4. Brief Description of the Figures and Tables

**Fig.1:** Inhibition binding of 425 to sEGFR by 5A6, 3B6, 15H8 or unrelated mAbs Percentaje of inhibition is calculated as described in material and methods using OD values from ELISA.

**Fig.2 :** Inhibition binding curves of murine (A) and humanized version (B) of 425 to EGFR expressing A431 Cells by 5A6(●), 3B6 (○), 15H8 (▽) and murine IgG1(Sigma) (▼). Results are expressed as OD mean of duplicate samples. x-ordinate: competitor (µg/ml), y-ordinate: OD.

**Fig.3:** Competitive ELISA for Ab3-Ab2 binding. Serial dilutions of rat Ab3 sera were tested for binding to solid-phase coated Ab2 or unrelated mab15, previously incubated with 5A6 (●), 3B6(■) and 15H8(▲). mAb15(▽) and Normal Mouse sera (♦) were used as controls. *A*: rat Ab3 sera anti-5A6; *B:* rat Ab3 sera anti-3B6; *C*: rat Ab3 sera anti-15H8; *D*: rat control sera anti-mAb15; y-ordinate: OD, x-ordinate: serum dilutions x $10^{-3}$.

**Fig.4:** Solid phase ELISA: Inhibition binding of Balb/c and B6D2F1 Ab3 from 5A6 and 3B6 immunized mice to purified EGFR by Anti-Ids.

**Fig.5:** Complete sequence of variable region (including leader sequence) of heavy and light chain of 15H8, 5A6 and 3B6 mouse monoclonal antibodies. The nucleotide and amino acid sequences are shown.The leader sequence if annotated in bold, CDR sequences are in italic and the sequence of the primers used for the amplification of the variable regions are underlined. The heavy chain of 15H8, 5A6 and 3B6 have the characteristic structure of group III D. The light chain of 15H8 and 5A6 have the characteristic structure of kappa group V, and 3B6 has the structure of kappa group III, both according to Kabat classification.

A: 15H8 heavy chain;      B: 15H8 light chain
C: 3B6 heavy chain;      D: 3B6 light chain
E: 5A6 heavy chain;      F: 5A6 light chain

**Fig.6:** Compared sequences of CDR2H, CDR3H and CDR2L of Ab2 and EGFR external domain. Identical amino acid are shown in closed boxes. Open boxes indicate similar amino acids. The potentially antigenic regions are shown in shaded boxes.

**Table I.** Affinity constants of 5A6, 3B6 and 15H8 for 425. Dissociation constants have been calculated by scatchard plots of binding of Ab2 to 425 from OD values obtained by ELISA performed as described in material and methods.

**Table II.** Ab1-Ab2 inhibition binding by Ab3 and detection of cross reacting idiotypes on Ab2. Twofold dilutions of Ab3 sera from mice (A), rabbits(B) and rats (C) immunized by Anti-Ids or unrelated mAbs were tested for binding inhibition of their immunizing Ab2, the other Anti-Ids and murine isotype and allotype matched mAbs. Serum titres showing 100% inhibition are given.

**Table III.** Anti-EGFR response in Balb/c mice determined by indirect immunofluorescence against EGFR positive (A431) or negative (C33A) unfixed cells, by ELISA against whole cells or purified external domain.

## 5. Detailed Description

### Biological Materials and General Methods

Microorganisms, cell lines, plasmids, phagemids, promoters, resistance markers, replication origins or other fragments of vectors which are mentioned in this application are commercially or otherwise generally available. Provided that no other information in the application is given, they are used only as examples and are not essential according to the invention and can be replaced by other suitable tools and biological materials, respectively.

The techniques which are essential according to the invention are described in detail in the specification. Other techniques which are not described in detail correspond to known standard methods which are well known to a person skilled in the art, or are described more in detail in the cited references and patent applications and in the standard literature (e.g. Harlow,E., and D.Lane.1988. *Antibodies,* a *laboratory manual.* Cold Spring Harbor Laboratory, N.Y.)

Balb/c mice (IFFA CREDO), B6D2F1 hybrid strain from C57BL/6J and DBA/2J mice (IFFA CREDO), Wistar Rats (Interfauna Ibérica) and New Zealand White Rabbits (Biocentre) were used for in vivo assays.

A431 is an epidermoid carcinoma (ATCC CRL1555) expressing EGFR. C33A (ATCC HTB31), a cervical carcinoma, is receptor negative. HL1-Friendly Myeloma-653 (Ventrex, Bioventures group) was used as fusion partner for hybridoma obtention. All lines were cultured in RPMI 1640 Medium supplemented with 10% Fetal Calf Serum and 2mM glutamine.

Anti-EGFR mAb 425 (Ab1) was generated according to Rodeck et al. (l.c.). Several murine mAbs of unrelated specificities were used as controls in the different assays: mab15 (IgG1,k), anti-gp85/45 of small cell lung carcinomas; R24, (IgG3,k), anti-GD3; 14F9 (IgG3,k), anti-GD3 and Me361 (IgG2a), anti-GD2 have been previously described (Kamma, H. et al.,1989, *Cancer Res. 49(8): 5118;* Dippold, W.G. et al., 1980, *Proc. Natl. Acad. Sci. USA. 77:6114;* Massó, O. et al.,1991, *Immunología 10:36;* Thurin, J. et al., 1987, *Cancer Res. 47:1229*).

F111 (IgG1,k) was obtained by PEG-induced fusion between splenocytes from RNase immunized Balb/c mice and Friendly-Myeloma as described below.

### Anti-425 Idiotype mAb obtention.

Murine hybridomas have been generated using splenocytes from Balb/c mice immunized with mAb 425 and fused with 653-Friendly Myeloma cells. Supernatants were tested by sandwich ELISA against mAb 425. 5A6, 3B6 and 15H8 hybridomas were obtained from two fusions that gave 2,8% and 10,5% specific efficiency respectively. After cloning three times by the limiting dilution method, they were found to be IgG1,k.

### 5A6, 3B6 and 15H8 characterization.

Crude supernatants or protein A purified samples were used for Ab2 characterization. The Anti-Id nature was confirmed by their specific binding to mAb 425, when tested against a panel of non-related mAbs with different antigenic specificities. (F111, 361, 15 and 14F9).

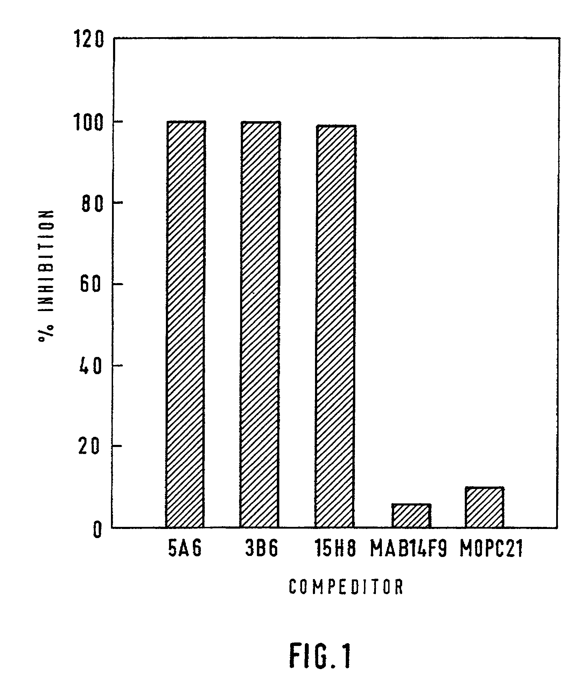

To define the localization of 425 idiotopes recognized by anti-Ids, they were tested for 425-EGFR inhibition binding by ELISA against sEGFR. All them completely inhibited its reactivity (Fig. 1), indicating that recognize idiotopes within the antigen binding site.

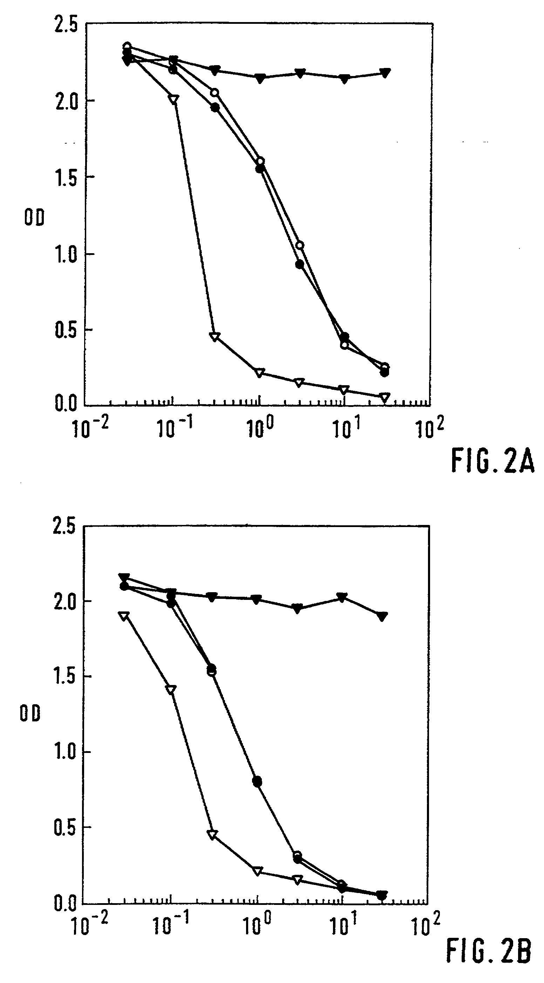

To define 5A6, 3B6 and 15H8 Anti-Ids as Ab2b we determined whether the idiotopes recognized on mAb 425 coincide with the CDRs or antigen-binding site. For these analysis comparative inhibition-binding curves were established with the murine (Fig.2A) and humanized (Fig.2B) version of 425 mAb by indirect ELISA using A431 cells. All three Anti-Ids can inhibit binding of both versions of 425, each Ab2 has similar inhibition-binding curves showing that the 425 idiotopes recognized by 5A6, 3B6 and 15H8 are directly implicated in antigen recognition. 15H8 was 10 times more effective to inhibit 425-A431 cells binding than 5A6 and 3B6. The Dissociation constant (KD) of Anti-Ids was calculated to determine the degree of fit of Ab2 to 425 paratope, KD constant of each Ab2 is given in Table I, 15H8 has more affinity with 425 correlating with its higher capacity for inhibit EGFR-425 binding. These results also suggest that at least two different idiotopes on mAb425 are recognized by Anti-Ids.

Table I

| Ab2 | KD |
|------|-----|
| 5A6 | $1.002 \ 10^{-9}$M |
| 3B6 | $1.25 \ 10^{-9}$M |
| 15H8 | $0.3 \ 10^{-10}$M |

***Syngeneic (Balb/c), allogeneic (B6D2F1) and xenogeneic ( rat, rabbit) Ab3 induction.***

The usefulness of this Ab2 as Id vaccines has been evaluated analyzing their immunogenicity. True internal image Ab2 should be able to induce Ab3 with Ab1-like specificity crossing the species barrier. Ab3 were generated in mice, rats and rabbits following immunizing protocols described above. Anti-anti-Id antibodies were detectable 2 weeks after the first dose and reached the maximum level 6-8 weeks after the first dose was given. Specific response against immunizing Ab2 was maintained for up to 12 months in rats and rabbits while it was transient in mice decreasing Ab3 titre in the next months. All rabbits generated Anti-murine Igs; Ab3 sera titre against immunizing Ab2 was 5-10 times higher than those found against isotype and allotype matched Igs, indicating that the immune response against the constant region of Ab2 does not disturb the immune response against V region.

To determine an effective immunization against those Ab2 determinants which make contact with mAb 425 paratope (where the antigen-mimicking idiotopes should be located), an inhibition binding assay was performed in which the binding of 5A6, 3B6 or 15H8 Ab2 to mAb 425 was measured in presence of serial dilutions of Ab3 sera from animals immunized with Ab2 or unrelated murine mAbs. In Table II, the Ab3 sera dilution which produces 100% Ab2-Ab1 binding inhibition is given from Balb/c mice (A), rabbits (B) and rats (C). As shown in Table II A,B and C, all anti-5A6 Ab3 sera raised in the 3 species inhibited 5A6 and 3B6 mab-425 binding with the same efficiency, Anti-3B6 sera showed similar inhibition patterns suggesting that both anti-Ids may have shared idiotopes. Furthermore, some rat and murine Anti-15H8 sera were found to inhibit 5A6 and 3B6 to 425 binding suggesting a partial idiotypic identity. Such reactivities were not detected in Anti-15H8 rabbit sera suggesting that Ab3 response in rabbits can be mainly directed against other more immunodominant idiotopes, this possibility is also supported by the finding of higher inhibiting titres in rabbit Ab3 sera than those found in rats and mice.

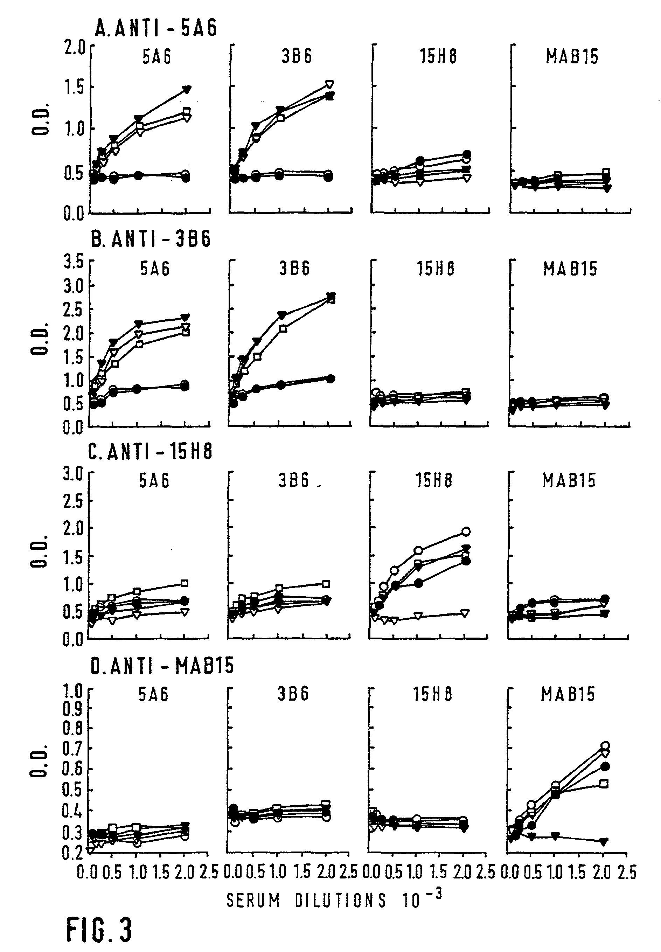

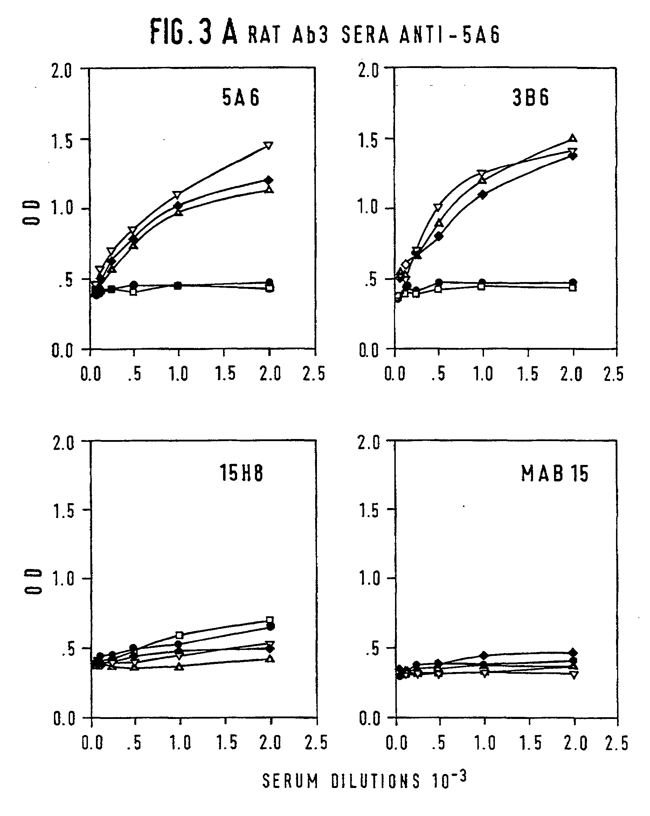

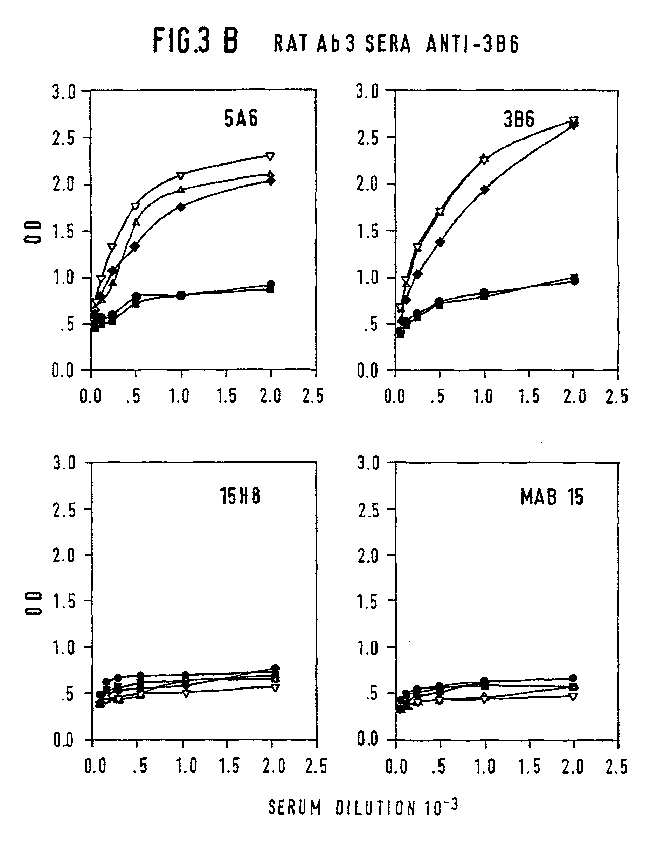

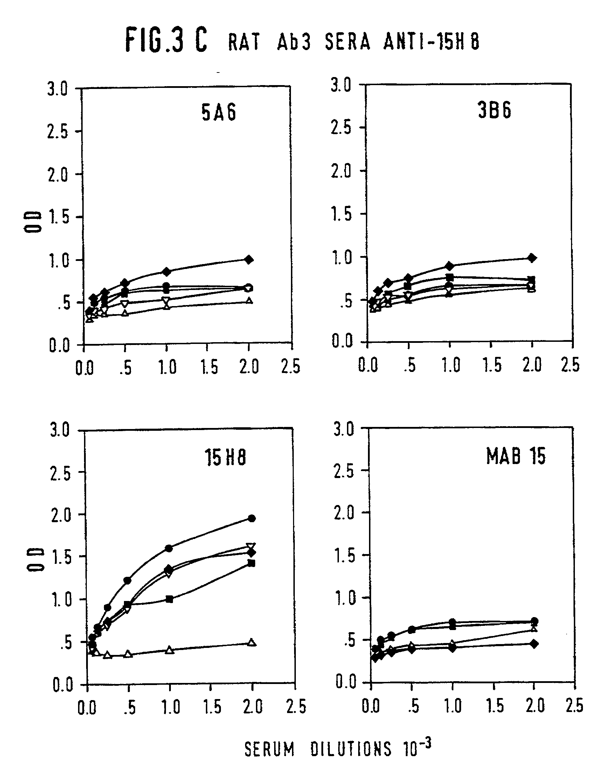

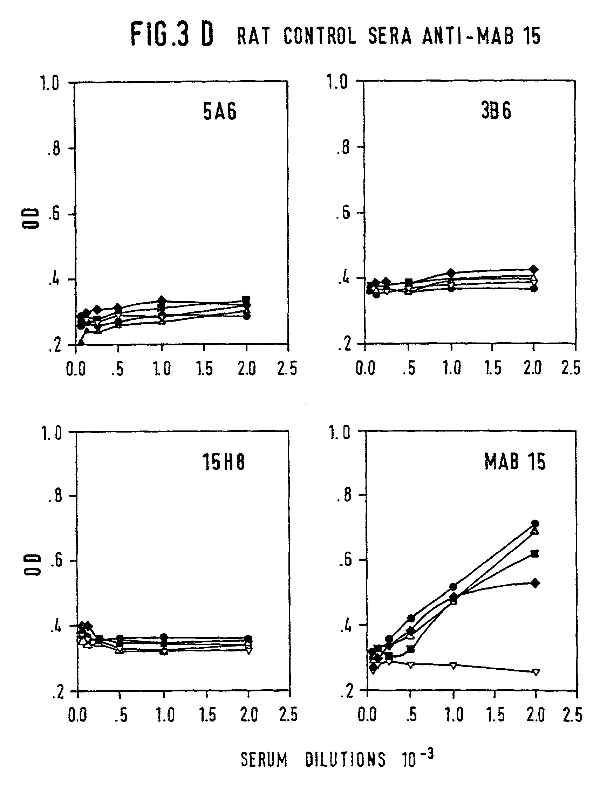

The antigenic identity between 5A6 and 3B6 was studied in a competitive ELISA, serial dilutions ($10^{-3}$ to $10^{-5}$) of rabbit and rat Ab3 sera were tested for Ab2 binding in presence of an equal volume (1mg/ml) of Ab2 or control mAbs. Inhibition binding curves corresponding to rat Ab3 sera are shown in Fig3. 5A6 and 3B6 generate identical Ab3 repertoire in rats and rabbits: Anti-5A6 sera reacts equally well with 5A6 or 3B6 Ab2 coated on ELISA plates and are equally inhibited by both Ab2. Rabbit Ab3 sera had similar pattern of inhibition to those shown if fig3. for rat sera.

Table II A

| Balb/c Ab3 | Ab2-mAB 425 inhibition Binding | | |
|------------|---------|---------|---------|
|  | 5A6 | 3B6 | 15H8 |
| 5A6 | 1/600 | 1/600 | - |
|  | 1/1200 | 1/1200 | 1/150 |
|  | 1/300 | 1/300 | - |
|  | 1/4800 | 1/2400 | 1/75 |
|  | 1/4800 | 1/4800 | 1/150 |
|  |  |  |  |
| 3B6 | 1/6400 | 1/6400 | - |
|  | 1/2400 | 1/2400 | - |
|  | 1/4800 | 1/4800 | - |
|  | 1/800 | 1/800 | - |
|  | 1/4800 | 1/4800 | - |

Table II A   (continued)

| Balb/c Ab3 | Ab2-mAB 425 inhibition Binding | | |
|---|---|---|---|
| | 5A6 | 3B6 | 15H8 |
| 15H8 | - | - | 1/9600 |
| | - | - | 1/9600 |
| | - | - | 1/9600 |
| | - | - | 1/9600 |
| | 1/2400 | 1/2400 | 1/9600 |
| F111 | - | - | - |
| | - | - | - |
| KLH | - | - | - |
| | - | - | - |
| | - | - | - |
| Preinmune sera | - | - | - |
| | - | - | - |

Table II B

| Rabbit Ab3 | Ab2-mAb 425 inhibition binding | | |
|---|---|---|---|
| | 5A6 | 3B6 | 15H8 |
| 5A6 | 1/3200 | 1/3200 | - |
| | 1/12800 | 1/12800 | - |
| | 1/6400 | 1/12800 | - |
| | 1/12800 | 1/6400 | - |
| | 1/12800 | 1/12800 | - |
| 3B6 | | | |
| | 1/6400 | 1/6400 | - |
| | 1/3200 | 1/6400 | - |
| | 1/12800 | 1/12800 | - |
| | 1/12800 | 1/12800 | - |
| | 1/12800 | 1/12800 | - |
| 15H8 | | | |
| | - | - | 1/12800 |
| | - | - | 1/12800 |
| | - | - | 1/1600 |
| | - | - | 1/3200 |
| mAb15 | - | - | N.D |
| | - | - | - |
| F111 | - | - | - |
| | - | - | - |
| mAb425 | - | - | - |
| | - | - | - |

Table II B   (continued)

| Rabbit Ab3 | Ab2-mAb 425 inhibition binding | | |
|---|---|---|---|
| | 5A6 | 3B6 | 15H8 |
| KLH | - | - | - |
| | - | - | - |
| Preinmune-sera | - | - | - |
| | - | - | - |

Table II C

| Rat Ab3 | Ab2-mAB 425 inhibition binding | | |
|---|---|---|---|
| | 5A6 | 3B6 | 15H8 |
| 5A6 | 1/2400 | 1/4800 | - |
| | 1/2400 | 1/2400 | - |
| 3B6 | 1/2560 | 1/2560 | - |
| | 1/1280 | 1/2560 | - |
| 15H8 | 1/160 | 1/320 | 1/2560 |
| | - | - | 1/1280 |
| F111 | - | - | - |
| | - | - | - |
| MAB 15 | - | - | - |
| | - | - | - |
| KLH | - | - | - |
| | - | - | - |
| | - | - | - |
| Preinmune-sera | - | - | - |
| | - | - | - |

## Identification of Anti-EGFR Ab3 antibodies.

Anti receptor antibodies were detected in Ab3 sera by direct immunofluorescence against A431 and C33A cells, and results are shown in Table III:

Table III

| Ab3[c] | Immunofluorescence | | ELISA | |
|---|---|---|---|---|
| | A431 | C33A[a] | A431 | sEGFr[b] |
| Anti-5A6 | 7/14 | 0/14 | 6/14 | 5/15 |
| Anti-3B6 | 10/13 | 0/13 | 6/13 | 5/16 |
| Anti-15H8 | 4/12 | 0/12 | 0/12 | 0/11 |
| Anti-mAb15 | 0/2 | 0/2 | 0/2 | 0/4 |
| Anti-R24 | 0/3 | 0/3 | 0/3 | 0/3 |

[a])Detection with FITC rabbitAnti-murine IgM+IgG.
[b])Detection with FITC rabbit Anti-murine IgG
[c])Positive animals/immunized animals.

EP 0 745 612 B1

Table III   (continued)

| Ab3[c] | Immunofluorescence | | ELISA | |
|---|---|---|---|---|
| | A431 | C33A[a] | A431 | sEGFr[b] |
| KLH | 0/5 | 0/5 | 0/5 | 0/4 |
| Preimmune | 0/20 | 0/20 | 0/20 | NT |

[a] Detection with FITC rabbitAnti-murine IgM+IgG.

[b] Detection with FITC rabbit Anti-murine IgG

[c] Positive animals/immunized animals.

Anti-Id vaccination in murine system induces the formation of Anti-EGFR antibodies, however no Anti-EGFR antibodies could be detected in Ab3 from rats and rabbits. The development of Anti-EGFR antibodies in B6D2F1 mice confirms that Ab2 vaccination is not genetically restricted to murine Balb/c strain. Specific antibodies reached titres 1/50-1/1600 when tested against sEGFR by ELISA.

### Ab3-EGFR inhibition binding by 5A6, 3B6 and 15H8.

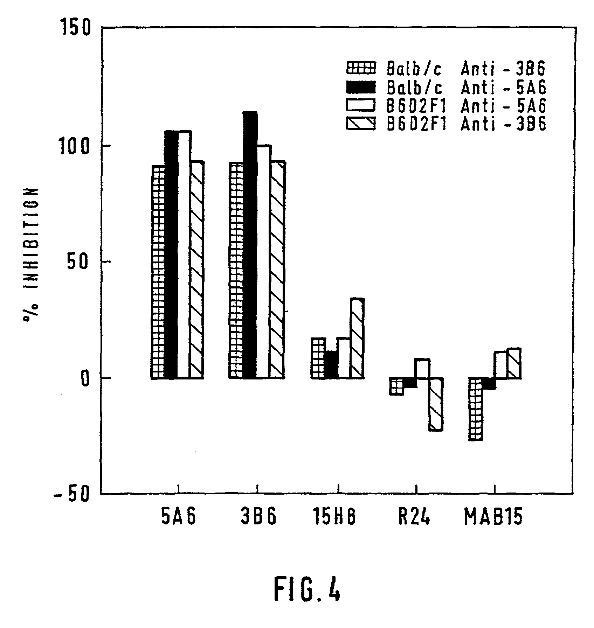

These assays were performed to test if Anti-Ids mimick in a three-dimensional fashion the EGFR. Different dilutions of Anti-EGFR Ab3 sera from Balb/c and B6D2F1 mice were mixed with Anbti-Ids and left react 4 hours room temperature, then, they were tested for anti-receptor antibodies with sEGFR coated plates and the percentage of inhibition calculated from OD values is shown in Fig.4. 5A6 and 3B6 gives 100% inhibition indicating that share the three-dimensional structure of the original antigen. 15H8 gave 15-35% inhibition of Anti-EGFR Ab3 from 5A6 or 3B6.

It has been claimed that true internal image Anti-Ids can subtitute the immunogenic properties of antigen and induce specific response in different species, this is not the case of 5A6 and 3B6 that fails to induce Anti-EGFR in rats and rabbits; the serologic assays performed suggest, however, that the anti-receptor antibodies detected in mice are induced by 5A6 and 3B6 idiotype determinants resembling EGFR.

### Primary structures of Ab2 and comparison of amino acid sequences with EGFR.

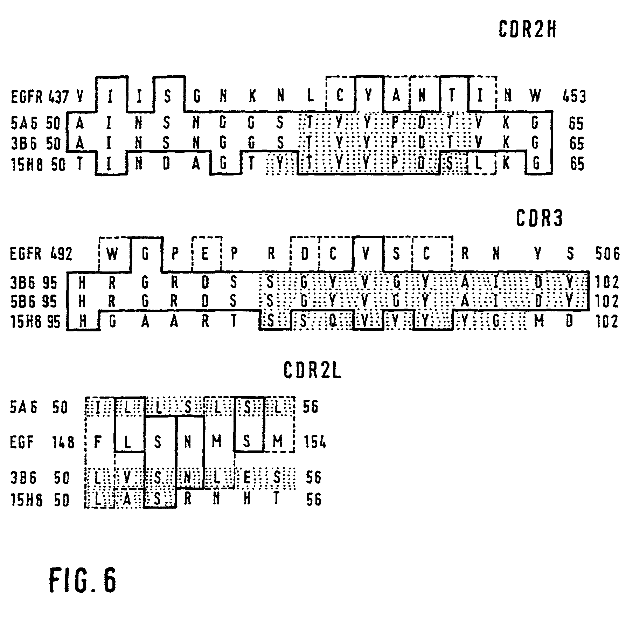

DNA sequencing has been performed to analyze the structural basis of their immunogenic properties. The VH and VL sequences of 5A6, 3B6 and 15H8 are displayed in Fig.5. The amino acid sequences of VH regions were classified into subgroup IIID according to Kabat. VL regions of 5A6 and 15H8 belong to kV group and 3B6 to kIII according to Kabat. CDR sequences were compared with amino acid sequences of human EGFR (GQHUE from SPIRS data bank), (Fig. 6) are shown in closed boxes the identic residues found and open boxes those which are equivalent. Maximun homology was found between residues 77-84 from CDR2H and residues 125-129 from CDR3H and 70-76 from 5A6 and 74-80 CDR2L, the highest homologous EGFR regions, amino acid 437-452 and 492-502 with CDR3H are located in the hypothetical ligand-binding domains of receptor protein described by A. Ullrich et al. (30). 15H8 shares identical CDR2H sequence but has different CDR3H and CDR2L sequences. To analyze which role plays each homologous CDR in the formation of internal image of EGFR, we have compared 5A6 and 3B6 sequences with known VH and VL regions compiled by Kabat. Some of the VH chains according to the invention are found to be highly homologous to 5A6 and 3B6, they show similar CDR1H and CDR2H, suggesting that CDR3H sequences play the main role in 425-paratope recognition and EGFR mimicry. It was found that there are 63 Igs (mainly from murine of human origin) with CDR3H regions partially homologous (50-87%) to CDR3H from 5A6 and 3B6. Homologous amino acid appear accumulated at residues 100H-102 while less homologous amino acid have been found along GYVG segment where the maximum homology with EGFR has been found. No antibody from murine or human origin has been found to posses the residue V at position 100D which is exclusive of 5A6, 3B6. VL 5A6 and 3B6 show homologies with murine Kappa chains, completely different VL chain sequences have the same specificity The role of CDR2L seems to be not related to a strict amino acid identity. 5A6, 3B6 have different sequences constructing an identical EGFR internal image structure, however amino acid homologies with EGFR appear accumulated in this region.

To evaluate its immunological effect as Id vaccines, we have analyzed the accessibility and antigenic properties of CDR2H, CDR3H and CDR2L regions. The antigenic index (a measure of antigenic probability) was calculated from ANTIGENIC program, the secondary structure was defined by DSSP program in order to determine the accessibility of the homologous sequences found.

Secondary and primary structure analysis reveals that these homologous residues are accessible and also coincide with regions showing positive antigenic index, therefore they can be directly implicated in the anti-internal image response. VL5A6 and VL3B6 chains present 7 non-identical antigenic regions and 11024 and 11603 angstroms of accessible surface protein respectively, able to constitute antigenic idiotopes, however both antibodies generate identical Ab3 response in mice, rabbits and rats. These results suggest that the Anti-Anti-Id response found in this system is exclusively directed to Id defined by VH chains of 5A6 and 3B6.

As the immunological and structural analysis of 5A6 and 3B6 Anti-Ids shows that they are the internal image of EGFR external domain, we have analyzed why fails to induce response in a second specie. The positive antigenic GYVGYAIDY CDR3H region carrying the EGFR equivalent GYVGY segments, were compared with CDR3H sequences from murine, rat and rabbit antibodies. A large group of murine antibodies have the sequence YAIDY, so among the predicted antigenic region on CDR3H, the residues GYVG are expected to define a private idiotope immunogenic in mice. No rabbit Ig has found to posses YAIDY sequence, being possible that rabbit Anti-Anti-Id response is directed against other idiotopes defined by larger antigenic regions, spoiling the anti-internal image response.

In summary, the present invention discloses the following:

In the active immunotherapy of cancer different aproaches have been used. Anti-idiotypic antibodies mimicking the immunological effect of tumor markers have been applied in cancer patients to obtain an specific anti-tumor response regulated by Id-Anti-Id interactions.

Anti-Id mAbs directed against mAb 425 have been obtained to develop specific vaccines for EGFR. Three antibodies (5A6, 3B6 and 15H8) have been selected recognizing combining-site associated idiotopes on mAb425 defined by their CDR sequences as demonstrated by inhibition binding of humanized version of 425 mAb to EGFR.

Immunological and serological assays have shown that 5A6 and 3B6 act as internal images inducing an specific humoral response in mice. Ab1-like antibodies from Ab3 sera binds to membrane-bound EGFR on A431 cells and reacts against sEGFR. The reaction is completely inhibited by both 5A6 and 3B6 Ab2 (independently of the Anti-Id used for the immunization), suggesting that 5A6 and 3B6 carry the same internal image of EGFR. Immunological data suggest partial idiotypic sharing with the internal image idiotype of 5A6 and 3B6 and 425 paratope-related Id from 15H8, serologic assays have shown partial inhibition binding of Anti-5A6 and Anti-3B6 murine Ab3 to Ab2 or EGFR in the presence of 15H8. Immunological studies have revealed the lack of biological effect: IgM antibodies (reacting with A431 cells but failing to recognize sEGFR) have been induced, however they are not inhibited by 15H8. These results suggest that unspecific clones have been activated during the immunization protocols.

The investigation of structural correlation between Id and antigen have been performed to know what structures in the Anti-Ids are implicated in the generation of Anti-EGFR response.

After sequencing 5A6, 3B6 and 15H8 and compare amino acid homologies with the EGFR, we have found a set of homologous and equivalent amino acid in the antigenic and accesible regions of CDR2H, CDR3H and CDR2L. These data are in agreement with several reports demostrating the presence of homologous sequences in the CDRs (or adjacent regions) from VL and VH chains that participate in the structural formation of internal image bearing idiotypes (31-33). The finding that different sequences in 5A6 and 3B6, constructs two antibodies with identical immunological effect, has allowed us to analize the role of CDRs and framework regions in the induction of Ab3 response and in the conformation of internal image of EGFR.

CDR2L, CDR2H and CDR3H from 5A6 and 3B6 are probably implicated in the construction of the internal image because maximum homologies and equivalent amino acid have been found with EGFR external domain in this regions. A three-dimensional mimicry seems play a main role as deduced by the following findings

a) CDR2L from 5A6 and 3B6 are different, but give rise identical Anti-EGFR. response.
b) Homologous residues from CDR2H have been found in non-related antibodies, so their participation in the internal image structure is not dependent of an exclusive linear identity.
c) The affinities found between CDRs and residues 148-154, 437-452 and 492-502 from EGFR are mainly based on equivalent amino acid. d) Equivalent amino acid accumulate in those CDRs but not in other regions.

The idiotypic analysis of several families of antibodies (Anti-PC, Anti-dextran, Anti-galactan, Anti-NIP antibodies) compiled by Davie et al (34) have shown that in an antigen driven system, the expression of Ids involve a minimun of amino acid from CDR2H, CDR3H and a pairing with specific L chains. Our immunologic and sequencing data suggest that we have generated an Ab1-Ab2-Ab3 Id. cascade restricted to a limited set of idiotypic regions defined by 425-paratope and Ab2-paratope. The most important feature has been to obtain two Ab2s, 5A6 and 3B6 showing identic immunologic properties (and thus idiotypic determinants) originated from identical VH chains paired to different VL chains.

The biological effectiveness of Anti-Id antibodies correlates with their affinity with the idiotypic repertoire in the receptor specie. The analysis of residues implicated in the formation of 5A6 and 3B6 internal image structure have shown them included in a larger antigenic region (residues 100F-101 in H chain) found to be homologous to other murine antibodies (from NBRF protein Database) but not in rabbits. Thus internal image structure determines an idiotype "seen" by the murine immune system but masked in a larger idiotypic region when administered in rabbits. This hypotesis is also supported by the immunological results: Anti-5A6 and Anti-3B6 paratope-related Ab3 sera titres are higher in rabbits than in mice. Homologous 100F-101 residues have been also found to present in human antibodies (from NBRF Database), providing a teorethical basis for the effectivity of internal image 5A6 and 3B6 in human system.

The effectiveness of internal image antiidiotypic antibodies as vaccines has been demonstrated in various animal models and in clinical trials. The obtention of anti-Ids recognizing Anti-EGF antibodies have been recently reported by Suarez (Suarez, E. et al., 1993, *Immunologia 12:122*), who obtained 4 non-internal image mAbs.

5A6 and 3B6 are the first Internal image Anti-Id mAbs reported mimicking the External Domain of human EGFR The biological characteristics of mAb 425, determines that 5A6 and 3B6 mimick the ligand-binding site of EGFR.

Serological studies, biological effect in animal models, and structural analysis suggest their potential value in human vaccination

## 6. Therapeutic and diagnostic use

The antibodies according to the invention can be administered to human patients for therapy. Therefore, it is an object of the invention to provide a pharmaceutical formulation comprising as active ingredient at least one antibody or antibody fragment as defined above and in the claims, associated with one or more pharmaceutically acceptable carrier, excipient or diluent therefore.

Typically the antibodies of this invention will be injected intravenously or parenterally. Generally, the dosage ranges for the administration of the antibodies fragments are large enough to produce the desired tumor suppressing and tumor lysing effect. The dosage will depend on age, condition, sex and extent of the disease in the patient and can vary from 0.1 mg/kg to 200 mg/kg, preferably from 0.1 mg/kg to 100 mg/kg/dose in one or more doses administered daily, for one or several days.

Preparations for parenteral administration includes sterile aqueous or nonaqueous solutions, suspensions, and emulsions. Examples of non-aqueous solvents are propylene glycol, polyethylene glycol, vegetable oils such as olive oils, and injectable organic esters such as ethyl oleate and other solvents known in the art which are suitable for these purposes. The antibodies of this invention can be used in a composition comprising a physiologically acceptable carrier. Examples of such suitable carriers are saline, PBS, Ringer's solution, or lactated Ringer's solution. Preservatives and other additives such as antibiotics, antioxidants, and chelating agents may also be present in the pharmaceutical formulations.

The antibody (or, optionally, the antibody fragment) can also be conjugated according to known methods to cytokines such as IL-2 in order to support their cytotoxicity.

The pharmaceutical formulations of the present invention are suitable for the treatment of all kinds of tumors, including melanomas, gliomas and carcinomas, as well as tumors of the circulating system and solid tumors. For diagnostic purposes the antibody can be conjugated, for example, to a radio-opaque dye or can be radiolabelled. A preferred labelling method is the Iodogen method. Preferably the antibody will be administered as $F(ab')_2$ or scFv fragments for diagnostic purposes. This provides superior results so that backround substraction is unnecessary.

### Examples:

### Example (1) Humanized mAb 425.

The Humanization of mAb 425 has been previously described (Kettleborough,C.A. et al.,1991, *Protein Eng. 4:773).* The reshaped form of mAb425 contains the murine CDRs grafted in human variable regions keeping the antigen binding site structure. It has been used to define more precisely the localization of 425 idiotypes recognized by the Ab2.

### Example (2) Production of Anti-idiotypic mAbs to Anti-EGFR 425 mAb.

Balb/c mice were primed with i.p. injection of 75 mg. of purified 425 mAb coupled to KLH (Sigma), polymerized with glutaraldehide and mixed with CFA (Difco). Animals were boosted at days 7, 14, 21, 28 and 38 with the same immunogen emulsified with IFA. Fusion was done 3 days after the last boost. Spleen cells from immunized mice where fused with HL1-Friendly Myeloma-653 using 1450 polyethylenglycol (Boehringer Mannheim), following a protocol previously described (Carceller, A.,1988,. *Sangre 33(3):220*). Fused cells were grown in 39% RPMI 1640, 39% Hybridoma Medium (Gibco), 20% Fetal Calf Serum and 2% HAT. Positive hybrids were cloned three times by limiting dilution.

### Example (3) Detection and analysis of Ab2 activity.

Anti-idiotypic antibodies were detected by sandwich ELISA with polystyrene microtiter plates (Nunc, Maxisorb) coated with 1mg./ml.of purified 425 mAb. Plates were blocked by 2% BSA-PBS and crude supernatants or diluted sera in 0,2% BSA-PBS were incubated overnight at 4°. After washing with PBS-0,05% Tween 20, biotinylated mAb 425, prepared by the method of Harlow and Lane (l.c.), was added for 1 h. 37°, plates were washed three times and HRPO-streptavidin (Dako) diluted 1/1000 in 2%BSA-PBS was added 3-3-5-5 tetramethyl benzidine (Sigma) solution was used as substrate. OD was measured at 450nm.

**Example (4) Dissociation Constant (KD) of 5A6, 3B6 and 15H8 Ab2 mAbs.**

This determination was done according to the method of Friguet (Friguet,B. et al., 1985, *J.Immunol.Methods 77:305*), briefly: serial dilutions of mAb425 ($2.10^{-8}$M to $2.10^{-11}$M) were incubated with fixed concentrations of Ab2 until equilibrium was reached. The concentration of free Ab2 was measured by indirect ELISA using microtiter plates coated with 1mg./ml. of mAb 425 and HRPO-Rabbit Anti-murine IgG1 (Zymed).

**Example (5) Purified human EGFR external domain (sEGFR).**

The purification of external domain of human EGFR and the immunoaffinity chromatography from EGFR secreted by A431 cells has been previously reported (Weber,W. et al., 1984, *J.Biol.Chem. 259 (23):14631*). It was used to confirm the specificity of Ab3 by indirect and competitive assays.

**Example (6) Assays for localization of idiotypes recognized in 425 mAb.**

To localize 425 idiotypes defined by 5A6, 386 and 15H8, they were tested for their ability to inhibit 425-EGFR binding. This analysis was first performed with purified sEGFR Crude supernatants and 50mg./ml. of purified Ab2 were mixed with 125ng. of mAb 425. After 4h. incubation at 37°C., 425 activity was tested by ELISA using microtiter plates coated with 2,5mg./ml. of sEGFR. Plates were washed three times with PBS-1% BSA and AP-rabbit Anti-mouse Igs (Dako) was added. 3,3,5,5, tetramethilbenzidine was used as substrate and OD at 405nm. was recorded in a Titertek Multiscan plate reader.

These analysis were complemented by comparing their inhibition binding curves of murine and reshaped 425: 100 ng./ml. of murine or reshaped 425 were mixed with equal volumes of increasing concentrations ($2.10^{-2}$M to $2.10^{1}$M ) of purified 5A6, 3B6, 15H8 Ab2s or unrelated murine IgG1 antibodies. Remaining activity was tested by indirect CellELISA against A431 cells, previously fixed in microtiter plate ($5.10^{5}$ / well) with 0,1% glutardaldehyde. After 60'incubation at 37°C, cells were washed 3 times with PBS-1%BSA-0,05% Tween 20. HRPO-Goat-Anti murine IgG2a (Dianova) and HRPO-Goat Anti-human IgG was used respectively to detect bound Ab1, orthophenyldiamide (Sigma) was used as substrate.

**Example (7) Ab3 generation in syngeneic (Balb/c mice), allogeneic (F6D2F1 mice) and xenogeneic ( Wistar rat and NZW rabbits) system and Ab3 detection.**

Purified Ab2s were coupled to KLH (Calbiochem) by glutaraldehyde (Sigma) and emulsified with CFA or IFA. Animals were administered at days 0, 15, 30 and 60. In some assays, additional boosters were performed 8-12 months later to study the stability of Ab3 response. Animals received at each dose 75mg (mice), 150mg (rats) and 300mg (rabbits) of total Ig by intraperitoneal inoculation (mice) or subcutaneously at different sites (rats and rabbits). Control groups received same doses of irrelevant KLH-coupled murine Igs or KLH alone.

Blood samples were collected at day 0 and 1 week after each boost. Serial serum dilutions (in PBS-0,15% dry milk) were added to microtiter plates coated previously with 1mg/ml. of Ab2 or other marine Igs. Bound anti-anti-ids were detected with biotinylated Ab2 (in murine Ab3 sera), HRPO Rabbit-Anti Rat Igs (Dako) deprived for cross-reactivity against marine Igs and HRPO Swine Anti-Rabbit Igs (Dako). Anti-isotypic and allotypic antibodies developed in rats and rabbits were eliminated with extensive adsorption of 1/50-1/100 diluted sera samples with marine IgG1 after incubation overnight at 4°.

**Example (8) Ab2-Ab1 inhibition binding by Ab3 sera.**

This assay was done to detect antibodies reacting with those Ab2 sites that make contact with mAb 425-paratope. Briefly, 8-16 ng./ml purified 5A6, 3B6, 15H8 and F111 were mixed with serial dilutions of Ab3 containing sera, after overnight incubation at 4°C, remaining Ab2 activity was detected by direct ELISA against 1mg./ml of 425 coated on polystyrene plates. Bound Ab2 was detected with PA-Rabbit Anti-mouse IgG1 (Zymed).

**Example (9) Determination of Ab2 idiotypic identity by mutual competition for Ab3 binding.**

To determine idiotypic identity between each anti-id antibody, Ab2 were tested for binding inhibition of Ab3 sera to Ab2-coated plates.Serial dilutions of rat Ab3 sera ($10^{-3}$ to $10^{-5}$) were tested for Ab2 binding in presence of an equal volume (1 mg./ml.) of Ab2 or control mAbs previously incubated overnight at 4°.

**Example (10) Detection of Anti-EGFR antibodies within Ab3 response.**

Murine, rat and rabbit Ab3 sera was tested for Ab1-like activity using CellELISA against A431 cells as described before, or by direct ELISA with sEGFR (2,5mg./ml.). HRPO Rabbit Anti-mouse Igs (Dako), Rabbit Anti-Rat Igs (Dako) and Swine Anti-Rabbit Igs (Dako) were used respectively as second antibody. Anti-receptor antibodies were also tested by direct immunofluorescence assay using unfixed, live A431 cells, cultured in Terasaki plates.

**Example (11) EGFR-Ab3 inhibition binding by 5A6, 3B6 and 15H8 Anti-Ids.**

Sharing determinants between receptor and Anti-Ids were tested by competitive assay. 120-240 mg./ml. of 5A6, 3B6, 15H8 or unrelated IgG1 were incubated overnight at 4°C with 1/25-1/150 dilutions of Ab3 Anti-EGFR sera and tested for Anti-receptor activity by ELISA, CellELISA and direct immunofluorescent assays. The percentage of inhibition was calculated as follows:

$$\% \text{ Inhibition} = 1 - \left( \frac{\text{OD with Ab2 - OD background}}{\text{OD without Ab2 - OD background}} \right) \text{-}100$$

**Example (12) RNA and cDNA preparation.**

Total RNA was isolated from 5A6, 3B6 and 15H8 hybridoma cell lines. PBS-washed cells were suspended in guanidinium thiocyanate solution and the RNA was isolated using a cesium chloride gradient ultracentrifugation method from Chirgwin (Chirgwin et al., 1979, Biochemistry 18, 5294). First-stranded cDNA was synthesized using a Pharmacia kit. The synthesis was done in 15 µl with 5 µg of RNA, during one hour at 37°C. First strand cDNA was used directly form amplification without previous cloning.

**Example (13) PCR amplification.**

A set of PCR primers were used to amplify copies of the mouse light chain variable regions and mouse heavy chain variable regions. The 5' primers, were designed to hybridize to the leader sequence. Primers were a mixture of 61 oligonucleotides, for the heavy chain variable domain, and 405 oligonucleotides, for the kappa chain variable domain. 3' primers were designed to anneal to the constant region. A specific IgG1 and kappa mouse primers were used.
For amplification, with a thermostable DNA polymerase, 25µl reaction mixture containing: 1µl of the cDNA-RNA hybrid, 250 nM of the appropriate 5' and 3' primers mixture, 200mM of each dNTP, 1mM MgCl$_2$ (for the light chain), 2mM MgCl$_2$ (for the heavy chain), and 1 U of Taq polymerase (Cetus), was overlaid with mineral oil and subjected to a touchdown starting with a 60° C until 55°C of annealing temperature. One tenth of the PCR reaction was run on a 1% agarose TAE gel electrophoresis and ethidium bromide-stained to visualize the resulting PCR products.

**Example (14) Molecular cloning and sequencing.**

1µl of each PCR-product was ligated into TA vector (Invitrogen, San Diego). The DNA ligation reactions, for VH and VL chain regions of each Ab2, were transformed by a Heat-Shock into competent TG1 *E.coli* cells. This created six DNA libraries, one from light variable regions and the others from heavy variable regions of each mAb. Colonies were selected on LB plates with 100µg/ml carbenicilin and some were toothepicked for further analysis. Positive colonies were detected by PCR screening or using plasmid digestion as described by Güssow (Güssow, D., Clackson T., 1989 T; Nucleic Acids Res.. 17: 4000). Double-stranded plasmidic DNA was prepared (Wizard preps, Promega Corp.) for sequencing from transformants. Dideoxy-nucleotide chain termination, following the methodology from Sanger et al. (Sanger, F. et al., 1977, Proc. Nat.Acad. Sci. U.S.A. 74: 5463) was carried out using Taq polimerase. Primers used in the sequencing reactions annealed in TA vector.

**Example (15) Computer analysis and sequence comparison.**

The software program DSSP (Sybil) was used to calculate the secondary structure of Ab2 and their accessible residues. ANTIGENIC was used to predict the antigenic regions using the method of Kolaskar and Tongaonkar (Kolaskar,A.S. et al.,1990, *FEBS-Lett 276(1-2):172*). Ab2 and EGFR homologies were defined by BESTFIT program using Gencore disk for symbol comparison table. Compared human EGFR sequence was those reported earlier (Ullrich et al., 1984, Nature 309:418). Ab2 were compared with other known Igs using the Protein Identification Resource (PIR) data bank compiled by National Biomedical Research Fundation (NBRF) and PROSIS program.

## Claims

1. Monoclonal anti-idiotypic antibody (Ab2) mimicking the internal image of EGFR antigen recognized by a corresponding murine, humanized or chimeric, monoclonal Ab1 idiotypic antibody **characterized in that** the idiotypic antibody is or derives from murine mAb 425 produced by the cell line deposited under ATCC HB 9629.

2. Monoclonal anti-idiotypic antibody according to claim 1 obtainable by immunisation an animal with a corresponding murine, humanized or chimeric, idiotypic antibody.

3. Monoclonal antibody according to claims 1 or 2 wherein the CDR regions and the FR regions of said antibody comprise an amino acid of Figure 5A-F.

4. Monoclonal antibody according to claims 1 or 2 comprising a nucleotide sequence of Figure 5A-F coding for the CDR and FR regions of said antibody.

5. Process for the preparation of an anti-idiotypic antibody as defined in one of the claims 1 to 3, characerized in that an animal is immunized with a corresponding idiotypic antibody, which binds to the idiotypic antigen directly by its CDR regions, the synthesized anti-idiotypic antibody, which binds to the idiotypes of the parent antibody is separated and purified by convenient methods.

6. Pharmaceutical composition comprising an anti-idiotypic monoclonal antibody as defined in one of the claims 1 to 3 and, optionally, a pharmaceutically acceptable carrier.

7. Use of an anti-idiotypic antibody as defined in one of the claims 1 to 3 for the manufacture of a drug directed against tumors.

## Patentansprüche

1. Monoklonaler anti-idiotypischer Antikörper (Ab2), der die zellinterne Domäne des EGFR-Antigens, das von einem entsprechenden monoklonalen Maus-, humanisierten oder Hybrid-Ab1-idiotypischen Antikörper erkannt wird, nachahmt, **dadurch gekennzeichnet, daß** der idiotypische Antikörper der von der unter ATCC HB 9629 hinterlegten Zellinie produzierten Maus-mAb 425 ist bzw. von diesem abstammt.

2. Monoklonaler anti-idiotypischer Antikörper nach Anspruch 1, der durch Immunisierung eines Tiers mit einem entsprechenden Maus-, humanisierten oder Hybrididiotypischen Antikörper gewonnen werden kann.

3. Monoklonaler Antikörper nach den Ansprüchen 1 oder 2, wobei die CDR-Regionen und die FR-Regionen dieses Antikörpers eine Aminosäure gemäß Abbildung 5A-F enthalten.

4. Monoklonaler Antikörper nach den Ansprüchen 1 oder 2 mit einer Nukleotidsequenz gemäß Abbildung 5A-F, die für die CDR- und FR-Region dieses Antikörpers codiert.

5. Verfahren zur Herstellung eines wie in einem der Ansprüche 1 bis 3 definierten anti-idiotypischen Antikörpers, **dadurch gekennzeichnet, daß** ein Tier mit einem entsprechenden idiotypischen Antikörper, der an das idiotypische Antigen direkt mit seinen CDR-Regionen bindet, immunisiert wird, und der hergestellte anti-idiotypische Antikörper, der an die Idiotypen des Ausgangsantikörpers bindet, mit geeigneten Verfahren abgetrennt und gereinigt wird.

6. Pharmazeutische Zusammensetzung mit einem wie in einem der Ansprüche 1 bis 3 definierten monoklonalen anti-idiotypischen Antikörper sowie gegebenenfalls einem pharmazeutisch unbedenklichen Träger.

7. Verwendung eines wie in einem der Ansprüche 1 bis 3 definierten anti-idiotypischen Antikörpers zur Herstellung eines Arzneimittels gegen Tumore.

**Revendications**

1. Anticorps monoclonal anti-idiotypique (Ac2) imitant l'image inteme de l'antigène EGFR reconnu par un anticorps idiotypique Ac1 murin, humanisé ou chimère correspondant, **caractérisé en ce que** l'anticorps idiotypique est ou est issu de l'AcM murin 425 produit par la lignée cellulaire déposée sous le n° ATCC HB 9629.

2. Anticorps monoclonal anti-idiotypique selon la revendication 1, pouvant être obtenu par immunisation d'un animal par un anticorps idiotypique murin, humanisé ou chimère correspondant.

3. Anticorps monoclonal selon la revendication 1 ou 2, dans lequel les régions CDR et les régions FR dudit anticorps comprennent un aminoacide de la figure 5A-F.

4. Anticorps monoclonal selon la revendication 1 ou 2, comprenant une séquence nucléotidique de la figure 5A-F, codant pour les régions CDR et FR dudit anticorps.

5. Procédé pour la production d'un anticorps anti-idiotypique tel que défini dans l'une des revendications 1 à 3, **caractérisé en ce qu'**on immunise un animal avec un anticorps idiotypique correspondant qui se fixe à l'antigène idiotypique directement par ses régions CDR, on sépare l'anticorps anti-idiotypique synthétisé, qui se fixe aux idiotypes de l'anticorps parent et on le purifie par des méthodes convenables.

6. Composition pharmaceutique comprenant un anticorps monoclonal anti-idiotypique tel que défini dans l'une des revendications 1 à 3 et, éventuellement, un véhicule pharmaceutiquement acceptable.

7. Utilisation d'un anticorps anti-idiotypique tel que défini dans l'une des revendications 1 3, pour la fabrication d'un médicament dirigé contre des tumeurs.

FIG.1

FIG.2A

FIG.2B

FIG.3

FIG.3 A RAT Ab3 SERA ANTI-5A6

SERUM DILUTIONS $10^{-3}$

FIG.3 B    RAT Ab3 SERA ANTI-3B6

SERUM DILUTION 10⁻³

FIG.3 C RAT Ab3 SERA ANTI-15H8

FIG.3 D  RAT CONTROL SERA ANTI-MAB 15

FIG. 4

Leader
ATG GAC TCC AGG CTC  AAT TTA GTT TTC CTT GTC CTT GTT TTG AAA GGT
Met Asp Ser Arg  Leu Asn Leu Val Phe Leu Val Leu Val Leu Lys Gly
           FR1
GTC CAG TGT GAA GTG CAA CTG GTG GAG TCT GGG GGA GGC TTA GTG AAG
Val  Gln Lys Glu Val  Gln Leu Val  Glu Ser Gly  Gly  Gly  Leu Val Lys

CCT GGA GGG TCC CTG AAA CTC TCC TGT GCA GCC TCT GGA TTC ACT TTC
Pro Gly Gly  Ser Leu Lys Leu Ser Cys Ala Ala  Ser Gly Phe Thr Phe

   CDR1              FR2
AGT *GAC TAT TAC  ATG TAT* TGG TTT CGC CAG CAT CCG GGA AAG AGG CTG
Ser *Asp Tyr Tyr  Met Tyr* Trp  Phe Arg Gln His Pro  Gly Lys  Arg Leu

            CDR2
GAG TGG GTC GCA *ACC ATT AGT GAT GCT GGT ACT TAC ACC TAC TAT CCA*
Glu Trp Val Ala *Thr Ile  Ser Asp Ala Gly  Thr Tyr  Thr Tyr Tyr Pro*

        FR3
*GAC AGT CTG AAG GGG* CGA TTC ACC ATC TCC AGA GAC AAT GCC AAG AAC
*Asp Ser Leu Lys Gly* Arg Phe Thr Ile  Ser Arg Asp Asn Ala Lys Asn

AAC CTG TAC CTC CAA ATG AGC AGT CTG AAG TCT GAG GAC ACA GCC ATG
Asn Leu Tyr Leu Gln Met Ser Ser Leu Lys Ser Glu Asp Thr Ala Met

       CDR3
TAT TTC TGT GCA AGA *GAC GGG GCA GCT CGG ACT TCG TCC CAG GTT TAT*
Tyr Phe Cys Ala Arg *Asp Gly  Ala  Ala  Arg Thr Ser  Ser Gln Val Tyr*

      FR4
*TAC TAT GGT ATG GAC TAC* TGG GGT CAA GGA ACC TCA GTC ACC GTC TCC
*Tyr Tyr Gly  Met Asp Tyr* Trp  Gly Gln Gly  Thr Ser Val Thr Val Ser

   Mouse constant IgG1
TCA GCC AAA ACG ACA CCC CCA TCT GTC TAT CCA TTC CCG GGT TCC
Ser Ala Lys Thr Thr Pro Pro  Ser Val  Tyr Pro Phe Pro Gly Ser

# FIG. 5A

Leader
ATG GGC TTC AAG ATG GAG TCA CAT ATT CAG GTC TTT GTA TTC GTG TTG
Met Gly Phe Lys Met Glu Ser His Ile Gln Val Phe Val Phe Val Leu

FR1
CTC TGG TTG TCT GGT GTT GAT GGA GAC ATT GTG ATG ACC CAG TCT CAA
Leu Trp Leu Ser Gly Val Asp Gly Asp Ile Val Met Thr Gln Ser Gln

CDR1
AAA TTC ATG TCC ACA TCA GTA GGA GAC AGG GTC AGC ATC ACC TGC *AAG*
Lys Phe Met Ser Thr Ser Val Gly Asp Arg Val Ser Ile Thr Cys *Lys*

FR 2
*GCC AGT CAG AAT GTT CGT ACT GCT GTA GCC* TGG TAT CAA CAG AAA CCA
*Ala Ser Gln Asn Val Arg Thr Ala Val Ala* Trp Tyr Gln Gln Lys Pro

CDR2
GGG CAG TCT CCT AAA GCA CTG ATT TAC *TTG GCA TCC AAC CGG CAC ACT*
Gly Gln Ser Pro Lys Ala Leu Ile Tyr *Leu Ala Ser Asn Arg His Thr*

FR 3
GGA GTC CCT GAT CGC TTC ACA GGC AGT GGA TCT GGG ACA GAT TTC ACT
Gly Val Pro Asp Arg Phe Thr Gly Ser Gly Ser Gly Thr Asp Phe Thr

CTC ACC ATT AGC AAT GTG CAA TCT GAA GAC CTG GCA GAT TAT TTC TGT
Leu Thr Ile Ser Asn Val Gln Ser Glu Asp Leu Ala Asp Tyr Phe Cys

CDR3                                          FR4
*CTG CAA CAT TGG AAT TAT CCT CTC ACG* TTC GGC TCG GGG ACA AAG TTG
*Leu Gln His Trp Asn Tyr Pro Leu Thr* Phe Gly Ser Gly Thr Lys Leu

Mouse constant kappa
GAA ATA AAA CGG GCT GAT GCT GCA CCA ACT GTA TCC ATC TTC CCA CCA
Glu Ile Lys Arg Ala Asp Ala Ala Pro Thr Val Ser Ile Phe Pro Pro

TCC ACC CGG
Ser Thr Arg

# FIG.5B

Leader
ATG GAC TCC AGG CTC AAT TTA GTT TTC CTT GTC CTT GTT TTA AAA GGT
Met Asp Ser Arg Leu Asn Leu Val Phe Leu Val Leu Val Leu Lys Gly
                FR1
GTC CTG TGT GAC GTG AAG CTC GTG GAG TCT GGG GGA GGC TTA GTG AAG
Val Leu Cys Asp Val Lys Leu Val Glu Ser Gly Gly Gly Leu Val Lys

CTT GGA GGG TCC CTG AAA CTC TCC TGT GCA GCC TCT GGA TTC ACT TTC
Leu Gly Gly Ser Leu Lys Leu Ser Cys Ala Ala Ser Gly Phe Thr Phe

        CDR 1              FR2
AGT AAC TAT TAC ATG TCT TGG GTT CGC CAG ACT CCA GAG AAG AGG CTG
Ser Asn Tyr Tyr Met Ser Trp Val Arg Gln Thr Pro Glu Lys Arg Leu

                CDR 2
GAG TTT GTC GCA GCC ATT AAT AGT AAT GGT GGT AGC ACC TAC TAT CCA
Glu Phe Val Ala Ala Ile Asn Ser Asn Gly Gly Ser Thr Tyr Tyr Pro

                FR3
GAC ACT GTG AAG GGC CGA TTC ACC ATC TCC AGA GAC AAT GCC AAG AAC
Asp Thr Val Lys Gly Arg Phe Thr Ile Ser Arg Asp Asn Ala Lys Asn

ACC CTG TAC CTG CAA ATG AGC AGT CTG AAG TCT GAG GAC ACA GCC TTG
Thr Leu Tyr Leu Gln Met Ser Ser Leu Lys Ser Glu Asp Thr Ala Leu

                CDR 3
TAT TAC TGT GCA AGA CAT CGG GGG AGG GAC AGC TCG GGC TAC GTA GGG
Tyr Tyr Cys Ala Arg His Arg Gly Arg Asp Ser Ser Gly Tyr Val Gly
                FR4
TAT TCT ATA GAC TAC TGG GGT CAA GGA ACC TCA GTC ACC GTC TCC TCA
Tyr Ala Ile Asp Tyr Trp Gly Gln Gly Thr Ser Val Thr Val Ser Ser

mouse constant IgG1
GCC AAA ACG ACA CCC CCA TCT GTC TAT CCA TTC CCG GGT TCC
Ala Lys Thr Thr Pro Pro Ser Val Tyr Pro Phe Pro Gly Ser

# FIG.5C

Leader
<u>ATG GAG TCA GAC ACA CTC CTG CTA TGG GT</u>A CTG CTG CTC TGG GTT CCA
Met Glu Ser Asp Thr Leu Leu Leu Trp Val Leu Leu Leu Trp Val Pro
                FR1
GGT TCC ACT GGT GAC ATT GTG CTG ACA CAG TCT CCT GCT TCC TTA GCT
Gly Ser Thr Gly Asp Ile Val Leu Thr Gln Ser Pro Ala Ser Leu Ala
                                      CDR1
GTA TCT CTG GGG CAG AGG GCC ACC ATC TCA TAC *AGG GCC AGC AAA AGT*
Val Ser Leu Gly Gln Arg Ala Thr Ile Ser Tyr *Arg Ala Ser Lys Ser*

                                    FR2
*GTC AGT ACA TCT GGC TAT AGT TAT ATG CAC* TGG AAC CAA CAG AAA CCA
*Val Ser Thr Ser Gly Tyr Ser Tyr Met His* Trp Asn Gln Gln Lys Pro

                              CDR2
GGA CAG CCA CCC AGA CTC CTC ATC TAT *CTT GTA TCC AAC CTA GAA TCT*
Gly Gln Pro Pro Arg Leu Leu Ile Tyr *Leu Val Ser Asn Leu Glu Ser*

FR3
GGG GTC CCT GCC AGG TTC AGT GGC AGT GGG TCT GGG ACA GAT TTC ACC
Gly Val Pro Ala Arg Phe Ser Gly Ser Gly Ser Gly Thr Asp Phe Thr

CTC AAC ATC CAT CCT GTG GAG GAG GAG GAT GCC TCA ACC TAT TAC TGT
Leu Asn Ile His Pro Val Glu Glu Glu Asp Ala Ser Thr Tyr Tyr Cys

CDR3                               FR4
*CAG CAC ATT AGG GAG GTC TAC ACG* TTC GGA GGG GGG ACC AAG CTG GAA
*Gln His Ile Arg Glu Val Tyr Thr* Phe Gly Gly Gly Thr Lys Leu Glu

        Mouse constant kappa
ATA AAA CGG GCT GAT GCT GCA CCA ACT GTA <u>TCC ATC TTC CCA CCA TCC</u>
Ile Lys Arg Ala Asp Ala Ala Pro Thr Val Ser Ile Phe Pro Pro Ser

<u>ACC CGG</u>
Thr Arg

# FIG. 5D

Leader
ATG GAC TTT GGG CTC AGC TTG ATT-TTC CTT GTC CTT GTT TTT AAA GGT
Met Asp Phe Gly Leu Ser Leu Ile Phe Leu Val Leu Val Phe Lys Gly

      FR1
GTC CTG TGT GAC GTG AAG CTC GTG GAG TCT GGG GGA GGC TTA GTG AAG
Val Leu Cys Asp Val Lys Leu Val Glu Ser Gly Gly Gly Leu Val Lys

CTT GGA GGG TCC CTG AAA CTA TCC TGT GCA GCC TCT GGA TTC ACT TTC
Leu Gly Gly Ser Leu Lys Leu Ser Cys Ala Ala Ser Gly Phe Thr Phe

   CDR1             FR2
AGT *AAC TAT TAC ATG TCT* TGG GTT CGC CAG ACT CCA GAG AAG AGG CTG
Ser *Asn Tyr Tyr Met Ser* Trp Val Arg Gln Thr Pro Glu Lys Arg Leu

        CDR2
GAG TTT GTC GCA *GCC ATT AAT AGT AAT GGT GGT AGC ACC TAC TAT CCA*
Glu Phe Val Ala *Ala Ile Asn Ser Asn Gly Gly Ser Thr Tyr Tyr Pro*

     FR3
*GAC ACT GTG AAG GGC* CGA TTC ACC ATC TCC AGA GAC AAT GCC AAG AAC
*Asp Thr Val Lys Gly* Arg Phe Thr Ile Ser Arg Asp Asn Ala Lys Asn

ACC CTG TAC CTG CAA ATG AGC AGT CTG AAG TCT GAG GAC ACA GCC TTG
Thr Leu Tyr Leu Gln Met Ser Ser Leu Lys Ser Glu Asp Thr Ala Leu

     CDR3
TAT TAC TGT GCA AGA *CAT CGG GGG AGG GAC AGC TCG GGC TAC GTA GGG*
Tyr Tyr Cys Ala Arg *His Arg Gly Arg Asp Ser Ser Gly Tyr Val Gly*

    FR4
*TAT GCT ATA GAC TAC* TGG GGT CAA GGA ACC TCA GTC ACC GTC TCC TCA
*Tyr Ala Ile Asp Tyr* Trp Gly Gln Gly Thr Ser Val Thr Val Ser Ser

mouse constant IgG1
GCC AAA ACG ACA CCC <u>CCA TCT GTC TAT CCA TTC CCG GGT TCC</u>
Ala Lys Thr Thr Pro Pro Ser Val Tyr Pro Phe Pro Gly Ser

# FIG. 5E

**Leader**
ATG GTG TCC ACA GCT CAG TTC CTT GTA TTT TTG CTT TTC TGG ATT CCA
Met Val Ser Thr Ala Gln Phe Leu Val Phe Leu Leu Phe Trp Ile Pro

FR1
GCC TCC AGA GGT GAC ATC TTG CTG ACT CAG TCT CCA GCC ATC CTG TCT
Ala Ser Arg Gly Asp Ile Leu Leu Thr Gln Ser Pro Ala Ile Leu Ser

CDR1
GTG AGT CCA GGA GAA AGA GTC AGT TTC TCC TGC *AGG GCC AGT CAG AGC*
Val Ser Pro Gly Glu Arg Val Ser Phe Ser Cys *Arg Ala Ser Gln Ser*

FR2
*ATT GGC ACA AGC ATA CAC* TGG TAT CAA CAA AGA ACA AAT GGT TCT CCA
*Ile Gly Thr Ser Ile His* Trp Tyr Gln Gln Arg Thr Asn Gly Ser Pro

CDR2                                      FR3
AGG CTT CTC ATA AGT *ATA CTT CTG AGT CTA TCT CTG* GGA GTC CCT TCC
Arg Leu Leu Ile Ser *Ile Leu Leu Ser Leu Ser Leu* Gly Val Pro Ser

AGG TTT AGT GGC AGT GGA TCA GGG ACA GAT TTT ACT CTT AGC ATC AAC
Arg Phe Ser Gly Ser Gly Ser Gly Thr Asp Phe Thr Leu Ser Ile Asn

CDR3
AGT GTG GAG TCT GAA GAT ATT GCA GAT TAT TAC TGT *CAA CAA AGT AAT*
Ser Val Glu Ser Glu Asp Ile Ala Asp Tyr Tyr Cys *Gln Gln Ser Asn*

FR4
*AGC TGG CCA TAC ACG* TTC GGA GGG GGG ACC AAG TTG GAA ATA AAA CGG
*Ser Trp Pro Tyr Thr* Phe Gly Gly Gly Thr Lys Leu Glu Ile Lys Arg

*Mouse constant kappa*
GCT GAT GCT GCA CCA ACT GTA TCC ATC TTC CCA CCA TCC ACC CGG
Ala Asp Ala Ala Pro Thr Val Ser Ile Phe Pro Pro Ser Thr Arg

# FIG. 5F

CDR2H

```
EGFR 437  V  I  I  S  G  N  K  N  L  C  Y  A  N  T  I  N  W  453
5A6  50   A  I  N  S  N  G  G  S  T  Y  Y  P  D  T  V  K  G  65
3B6  50   A  I  N  S  N  G  G  S  T  Y  Y  P  D  T  V  K  G  65
15H8 50   T  I  N  D  A  G  T  Y  T  Y  V  P  D  S  L  K  G  65
```

CDR3

```
EGFR 492  W  G  P  E  P  R  D  C  V  S  C  R  N  Y  S  506
3B6  95   H  R  G  R  D  S  S  G  Y  V  G  Y  A  I  D  Y  102
5B6  95   H  R  G  R  D  S  S  G  Y  V  G  Y  A  I  D  Y  102
15H8 95   H  G  A  A  R  T  S  S  Q  V  Y  Y  Y  G  M  D  102
```

CDR2L

```
5A6  50   I  L  L  S  L  S  L  56
EGF  148  F  L  S  N  M  S  M  154
3B6  50   L  V  S  N  L  E  S  56
15H8 50   L  A  S  R  N  H  T  56
```

FIG. 6